# EUROPEAN PATENT APPLICATION

(11) **EP 2 481 433 A2**
(43) Date of publication of application: **01.08.2012**
(21) Application number: 12161849.0
(22) Date of filing: 10.03.2009
(51) Int. Cl.: A61M 16/00, A61M 16/06

(54) **Helmet for artificial respiration without the aid of face masks or tracheal tubes, with improved wearability**

(30) Priority: 13.03.2008 IT MI20080427
(62) Divisional of application: 09154790.1
(71) Applicant: Dimar S.R.L., 41037 Mirandola (MO) (IT)
(72) Inventor: Borsari, Maurizio, 41037 MIRANDOLA (MO) (IT)
(74) Representative: Branca, Emanuela

(57) **Abstract**

A helmet for artificial respiration without the aid of face masks or tracheal tubes, with improved wearability comprises a body (12) for containing the head of the patient and a neck collar (13, 113) - for forming an airtight sealing around the patient's neck - made of plastic flexible material, a plurality of fittings (14) fixed onto the container body (12) for the connection of a ventilation apparatus, accessories (15) and for releasing air, the neck collar (13, 113) being directly connected to a lower end of the container body (12), wherein the container body (12) and the neck collar (13, 113) are entirely made of flexible material and thus free of rigid structural elements.

## Description

The present invention refers to a helmet for artificial respiration without the aid of face masks or tracheal tubes, with improved wearability.

Helmets for artificial respiration, comprising a container body made of plastic flexible material and provided with a neck collar applied to hold the head of the patient are currently used often - as an alternative to the face masks or tracheal tubes - in oxygen therapy and in the continuous positive pressure ventilation, the so-called CPAP, or in the Non-invasive ventilation, the so-called NIV.

The helmet can be connected to a ventilation machine and it is provided with at least one air outlet.

The abovementioned ventilation techniques are for example performed in the resuscitation units, intensive care units, pneumology, in the infectious diseases units, general medicine units, as well as in the hyperbaric chambers, as well as in home treatment.

The main problems concerning helmets of the known type regard the requirement of a plurality of sizes, the need of two people, i.e. four hands, for wearing and removing it, the wearability of the same on patients who, additionally, are in critical conditions. Furthermore, the helmet must be worn by the patient also for long periods of time both in sitting position and in a position lying on a bed.

The helmets must be fittable onto the head of the patient and fastenable around the neck of the same, as well as removable, in a quick manner and causing least discomfort possible to the patient who is already in pain.

Source of discomfort to the patient are the armpit straps, which are constrained to the helmet and hold it in position on the head of the patient operating against the pressure that would tend to raise it.

An object of the present invention is that of providing a helmet for artificial respiration without the aid of face masks or tracheal tubes, with improved wearability, capable of overcoming the abovementioned drawbacks of the prior art.

Another object of the present invention is that of providing a helmet for artificial respiration that can be worn even during the sleep in a lying down position, especially when treating diseases at the patient's home.

Another object of the present invention is that of providing a helmet for artificial respiration that can be fitted onto the head of the patient and subsequently removed in a simple and quick manner by only one operator or by the patient himself.

Further object of the present invention is that of providing a helmet for artificial respiration that can be fitted onto the head of the patient in a simple and quick manner.

Another object is that of providing a helmet for artificial respiration that allows eliminating the presence of armpit straps or reducing the traction on the same to ease the discomfort of the patient.

The objects according to the present invention are attained by providing a helmet for artificial respiration without the aid of face masks or tracheal tubes, with improved wearability as outlined in the independent claims.

Further characteristics are provided for in the dependent claims.

Characteristics and advantages of a helmet for artificial respiration without the aid of face masks or tracheal tubes, with improved wearability according to the present invention shall be clearer from the following exemplifying and non-limiting description, with reference to the attached schematic drawings wherein:
figure 1 is a perspective view of a helmet for artificial respiration without the aid of face masks or tracheal tubes, with improved wearability, without the rigid structural elements;
figure 2 shows the helmet of figure 1 in position on the head of the patient during the ventilation therapy;
figures 3 and 4 show a neck collar with sliding closure of the "coulisse" type for an artificial respiration helmet subject of the present invention, respectively in open and closed position;
figure 5 is a perspective view of a helmet for artificial respiration without the aid of face masks or tracheal tubes, with improved wearability, provided with the neck collar of figures 3 and 4 and provided with counterweights;
figure 6 is a perspective view of a helmet subject of the present invention provided with a plurality of hooks for applying counterweights.

Referring to the figures, shown is a helmet for artificial respiration without the aid of face masks or tracheal tubes, with improved wearability, indicated in its entirety with 10.

The helmet 10 comprises a body 12 for containing the head of the patient and a neck collar 13, for fastening around the patient's neck, made of plastic flexible material. Generally, the container body 12 comprises at least one transparent front element, not shown in the figures, to allow the patient to see through the helmet.

The helmet 10 further comprises a plurality of fittings 14 fixed onto the container body 12 for connecting a ventilation apparatus, not shown, and for connecting various accessories 15, such as for example valves, filters, caps or a compensation balloon. Furthermore, at least one of the fittings 14 allows the exit of the air from the helmet 10.

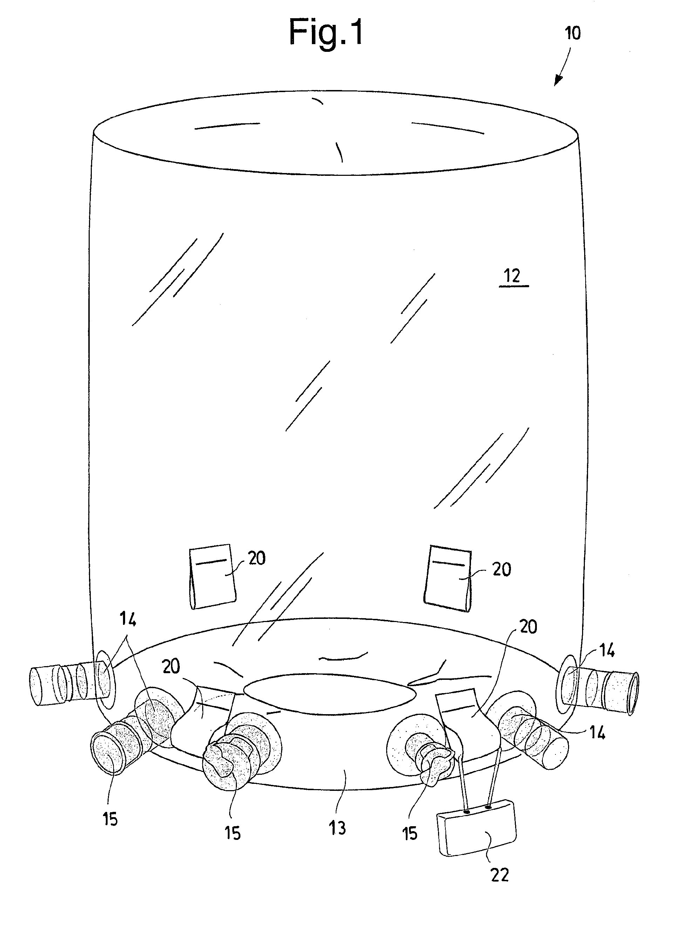

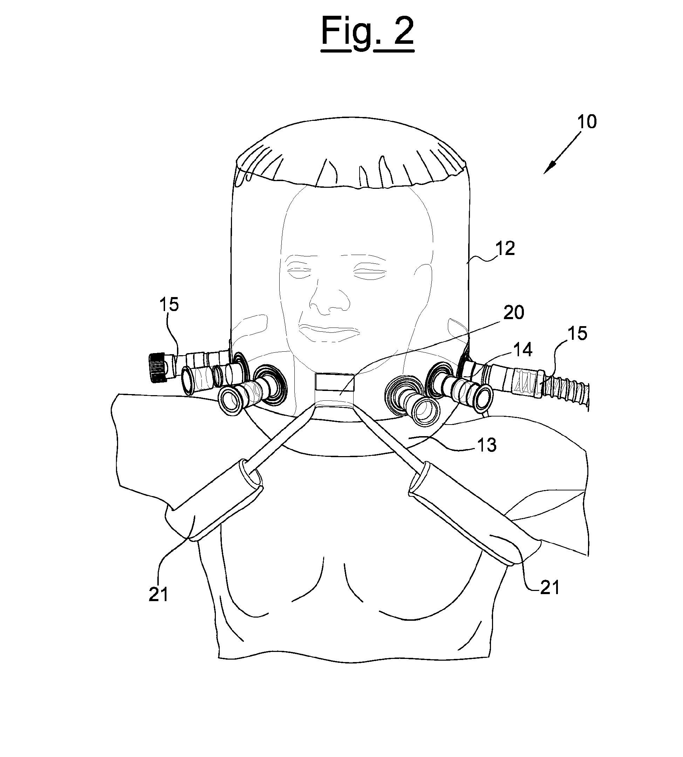

The helmet 10 shown in figure 1 and 2 is free of the rigid structural elements, and thus it does not have a rigid or semi-rigid ring. As a matter of fact, the neck collar 13 is directly connected to a lower end of the container body 12. In this manner, the container body 12 and the neck collar 13 are entirely made of flexible material and thus free of rigid structural elements.

This embodiment of the helmet 10 offers greater comfort for the patient, in particular if the helmet is to be worn for long periods of time and/or in a lying down position, such as when sleeping. This is for example the case when treating the so-called sleep apnea disorder, that is the apnea phenomena from which some patients suffer during the phase of deep sleep, or the REM sleep.

Eliminating the rigid or semi-rigid ring between the container body and the neck collar implies eliminating the risk of decubitus ulcers on the patient's nape and makes the helmet suitable even for newborns or patients affected by the Down's syndrome.

The container body 12 and the neck collar 13 may be alternatively made in a single piece of the same material, or be made of two different materials fixed to each other.

The neck collar 13 may be made of elastically yielding plastic material, generally polyurethane, in order to form an airtight seal around the patient's neck. Due to the effect of the positive pressure inside the helmet during the ventilation therapy, the neck collar swells on the patient's chest, acquiring the configuration of figure 2. The container body 12 is instead generally made of polyvinyl chloride.

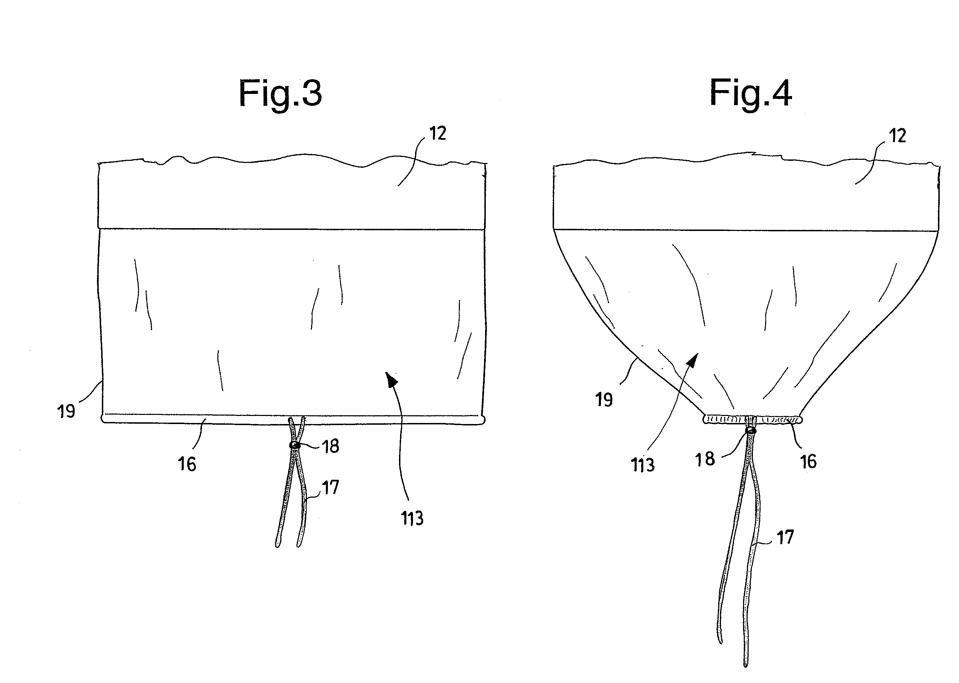

However, should polyvinyl chloride for example be used for the entire helmet, a neck collar 113 is advantageously provided with a sliding closure device, the so-called coulisse. For example, as shown in figures 3 - 5, the neck collar 113 comprises a substantially cylindrical sleeve 19 constrained at an upper end thereof to the container body 12 and provided with a lower end of the sliding closure device. The sliding closure device comprises a circumferential pocket 16 made by joining the lower edge of the sleeve 19, a cord 17 sliding into the pocket and a closure button 18 for firmly holding the two ends of the cord 17 in the desired position, and subsequently loosening this constraint.

As shown in figures 1 and 2, the container body comprises at least one pair of eyelets, or rings, 20 made of plastic material fixed at opposite ends of the circumference of the container body 12 for the connection of the fastening elements 21 to discharge the upward thrust to which the helmet is subjected due to the ventilation therapy.

In the example of figure 2, the fastening elements 21 are made up of two cushioned armpit straps, connected to a front ring 20 and to an opposite ring, not shown.

Shown in figure 1 instead are two pairs of rings 20 arranged at opposite points of the circumference of the container body 12.

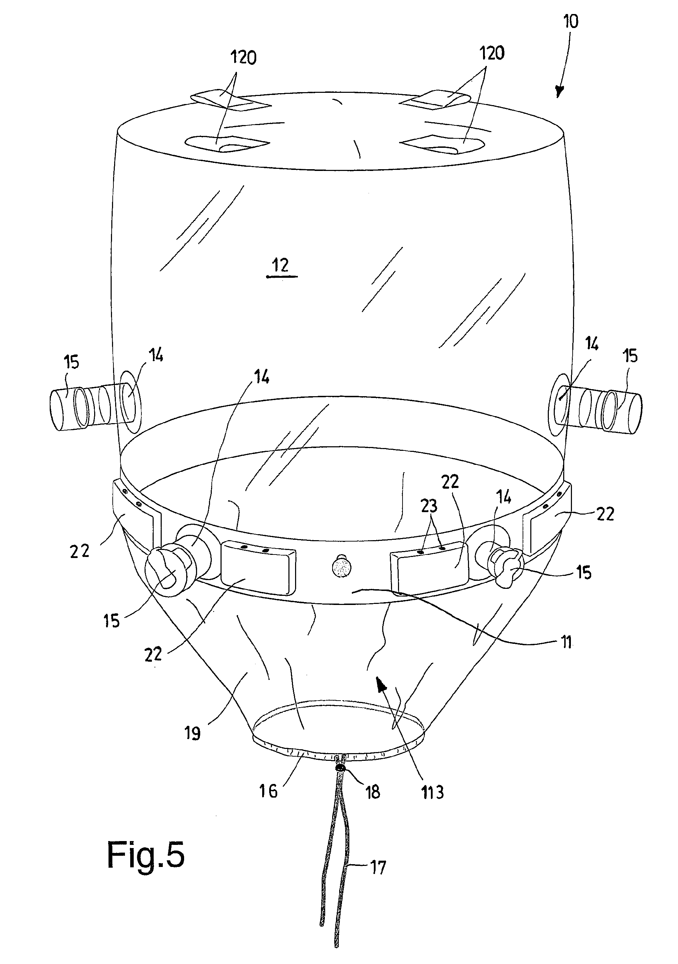

The helmet 10 free of rigid structural elements may also be provided with at least one pair of rings 120, fixed in an upper portion of the container body 12, as shown in figure 5, for the connection of the fastening elements 21.

The fastening elements 21 connected to the rings 20 or 120 to discharge the traction of the helmet, may also be connected to fixed points with respect to the patient, such as for example the bed rails, or also be constrained to counterweights 22 adapted to counteract the upwards thrust on the helmet, as schematically shown in figure 1.

Another important aspect of the helmet 10 according to the present invention is that the neck collar 113 provided with sliding closure, is suitable to be provided for on each type of helmet - also for example on a helmet provided with a rigid ring 11 between the container body and the neck collar, such as the one shown in figure 5, and regardless of the positioning - with other components such as rings 20 and 120 or counterweights 22 on the helmet.

The helmet 10 provided with neck collar 113 having a sliding closure, is suitable to be worn by any patient, without requiring providing for up to six different neck sizes. Furthermore, when the neck collar 113 is at the open position (figure 3) it may be fitted onto the head of the patient even by one person alone, such operation not being possible when using traditional helmets, which require to be widened and fitted by two people. Furthermore, it is possible to remove the helmet 10 in an equally practical and quick manner, a crucial aspect in cases where quick access to the patient is required for the safety of the same.

Another aspect that characterises the helmet 10 according to the present invention is the presence of at least one pair of eyelets, or rings, 120 made of plastic material, fixed onto the container body 12 in an upper portion for the connection of the fastening elements 21 which allow discharging the upward thrust, which can even reach 5-10 kg.

The rings 120 on the upper portion of the container body 12 may be applied to any of the types of helmets described, and possibly also additionally to the pairs of rings 20 arranged circumferentially on the container body 12.

The possibility of connecting the fastening elements 21 not only to the patient, but also directly to the fixed points, such as for example the bed rails, particularly by means of the rings 120 in upper position, represents a considerable improvement to the comfort of the patient who can be relieved, maybe even only for short periods of time, of the discomfort caused by the armpit straps, which can cause decubitus ulcers in the most critical cases.

Another aspect of the helmet 10 of the present invention which improves the wearability of the same, is the presence of means for constraining at least one counterweight 22 to counteract the upward thrust of the helmet.

Due to the counterweights 22, of suitably calibrated weight, the fastening elements 21 or the traction on the same may be considerably reduced entailing a noticeable advantage.

The constraint means and the related counterweights can be conceived for any type of the helmets described. Shown in figure 1 for exemplification purposes is a constraint means made up of a ring 20, on which a counterweight 22 is hung.

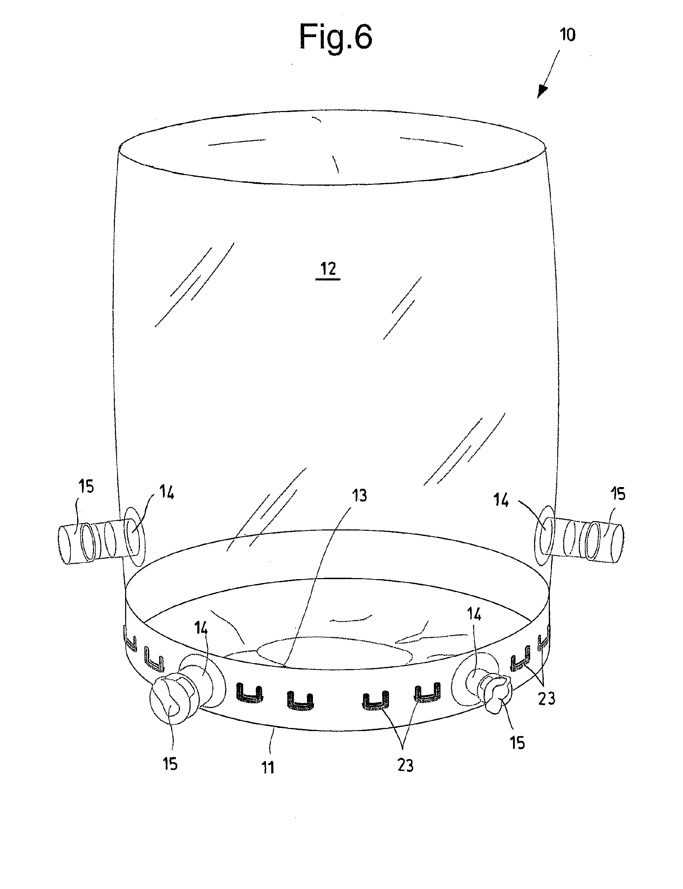

Shown in figure 6, instead, are constraint means comprising a hook 23 projecting outwards the helmet 10 and arranged on the structurally rigid ring 11 arranged between the container body 12 and the neck sealing collar 13. In the shown example, each hook 23 comprises a pair of parallel arms, for a firm positioning of a counterweight 22. According to further embodiments, not shown, the hooks may comprise more than two arms. Shown in figure 5 are four counterweights 22 fixed onto the hooks 23. The counterweights 22 may be made up of substantially parallelepiped plates provided with two through holes parallel and complementary to the parallel arms of the hooks 23.

The helmet for artificial respiration without the aid of face masks or tracheal tubes, with improved wearability subject of the present invention has the advantage of being comfortable for the patient even in presence of critical conditions. This makes it advantageously possible to use the helmet even for patients in determined critical conditions, in which the helmets are generally not useable.

Furthermore, the helmet for artificial respiration without the aid of face masks or tracheal tubes, with improved wearability may be conceived and supplied to the users in only one size, enhancing the warehouse management, and it can advantageously be fitted onto the head of the patient in an easy manner even by only one operator creating least discomfort to the patient.

The helmet for artificial respiration without the aid of face masks or tracheal tubes, with improved wearability thus conceived is susceptible to various modifications and variants, all falling within the invention; furthermore all details can be replaced by technically equivalent elements. In practice, the materials used, as well as the dimensions, may vary depending on the technical requirements.

## Claims

1. Helmet for artificial respiration without the aid of face masks or tracheal tubes, with improved wearability comprising a body (12) for containing the head of the patient and a neck collar (13, 113) - for forming an airtight sealing around the patient's neck - made of plastic flexible material, a plurality of fittings (14) fixed onto said container body (12) for the connection of a ventilation apparatus, accessories (15) and for releasing air, **characterised in that** said neck collar (13, 113) is directly connected to a lower end of said container body (12), the container body (12) and the neck collar (13, 113) being entirely made of flexible material and thus free of rigid structural elements, the helmet not having a rigid or semirigid ring.

2. Helmet for artificial respiration without the aid of face masks or tracheal tubes, with improved wearability comprising a body (12) for containing the head of the patient and a neck collar (113) - for forming an airtight sealing around the patient's neck - made of plastic flexible material, a plurality of fittings (14) for the connection of a ventilation apparatus, accessories (15) and for releasing air, **characterised in that** said neck collar (113) comprises a substantially cylindrical sleeve (19) constrained at an upper end to said container body (12) and provided at a lower end with a sliding closure device, or coulisse device, comprising a circumferential pocket (16), a cord (17) sliding into said pocket (16) and a button (18) for closing the ends of said cord (17).

3. Helmet for artificial respiration without the aid of face masks or tracheal tubes, with improved wearability comprising a body (12) for containing the head of the patient and a neck collar (13, 113) - for forming an airtight sealing around the patient's neck - made of plastic flexible material, a plurality of fittings (14) for the connection of a ventilation apparatus, accessories (15) and for releasing air, **characterised in that** said container body (12) comprises at least one pair of eyelets, or rings, (120) made of plastic material, said rings (120) being fixed onto an upper portion of said container body (12) for connecting the fastening elements (21) to discharge the upward thrust of the helmet.

4. Helmet for artificial respiration without the aid of face masks or tracheal tubes, with improved wearability comprising a body (12) for containing the head of the patient and a neck collar (13, 113) - for forming an airtight sealing around the patient's neck - made of plastic flexible material, a plurality of fittings (14) for the connection of a ventilation apparatus, accessories (15) and for releasing air, **characterised in that** it comprises means for constraining at least one counterweight (22) adapted to counteract the upward thrust on the helmet.

5. Helmet according to claim 4 **characterised in that** said constraint means are hooks (23) projecting outwards the helmet (10) and positioned on a structurally rigid ring (11) arranged between said container body (12) and said sealing neck collar (13, 113).

6. Helmet according to claim 5 **characterised in that** said hooks (23) comprise at least two parallel arms for the firm positioning of a counterweight (22).

7. Helmet according to any one of the preceding claims, **characterised in that** said container body (12) comprises at least one pair of eyelets, or rings, (20) made of plastic material fixed onto opposite points of the circumference of the container body (12) for connecting the fastening elements (21) to discharge the upward thrust of the helmet.

8. Helmet according to any one of the preceding claims, **characterised in that** said neck collar (13) is made of elastically yielding plastic material for forming an airtight sealing around the patient's neck.

9. Helmet according to any one of the preceding claims, **characterised in that** said container body (12) and said neck collar (13, 113) are made in a single piece made of the same material.
